# EUROPÄISCHE PATENTSCHRIFT

(11) **EP 2 745 781 B1**
(45) Veröffentlichungstag und Bekanntmachung des Hinweises auf die Patenterteilung: **15.03.2017**
(21) Anmeldenummer: 13197782.9
(22) Anmeldetag: 17.12.2013
(51) Int. Cl.: A61B 10/06, A61B 10/02

(54) **Endoskopisches Instrument für die retrograde Biopsie, insbesondere Synovialbiopsie**
Endoscopic instrument for retrograde biopsy, in particular synovial biopsy
Instrument endoscopique pour la biopsie rétrograde, en particulier la biopsie synoviale

(30) Priorität: 19.12.2012 DE 102012223788
(43) Veröffentlichungstag der Anmeldung: 25.06.2014
(73) Patentinhaber: Karl Storz GmbH & Co. KG, 78532 Tuttlingen (DE)
(72) Erfinder: Hügle, Thomas, 79100 Freiburg (DE); Fuchs, Alexander, 78256 Steißlingen (DE)
(74) Vertreter: RLTG

(56) Entgegenhaltungen:
- EP-A1- 2 241 273
- EP-A2- 0 513 471
- DE-A1-102009 010 520
- US-A- 5 320 110
- US-A- 5 569 299
- US-A1- 2011 237 975

## Beschreibung

Die vorliegende Erfindung betrifft ein endoskopisches Instrument für die retrograde Biopsie, insbesondere Synovialbiopsie, umfassend eine Kopfeinheit mit einem distalen Spitzenbereich zur Durchdringung von Gewebe, einem Maulteil mit einer Gewebeeingriffsformation zur Biopsatabtrennung, wobei das Maulteil von einer Ruhestellung in eine Arbeitsstellung in Richtung auf den distalen Spitzenbereich zu und zurück verschwenkbar ist, wobei in der Arbeitsstellung die Gewebeeingriffsformation im Wesentlichen von dem distalen Spitzenbereich weg orientiert ist, sowie einem im Bereich der Kopfeinheit zur Umgebung offenen ersten Fluidkanalbereich.

Ein endoskopisches Instrument zur Synovialbiopsie ist aus der DE 10 2009 010 520 A1 bekannt. Dieses bekannte enoskopische Instrument weist in seinem distalen Endbereich eine zur Durchdringung von Gewebe spitz ausgebildete Kopfeinheit auf, in welcher ein Maulteil verschwenkbar positioniert ist. In einer Ruhestellung ist das Maulteil im Wesentlichen in die Kopfeinheit zurückgeschwenkt, so dass das Eindringen der als Trokar wiksamen Kopfeinheit in Gewebe ermöglicht ist. Zur Synovialbiopsie wird die Kopfeinheit in eine von der Synovia umgrenzte Gelenkshöhle eingeführt. In einem in der Ruhestellung des Maulteils proximal an dieses anschließenden Bereich der Kopfeinheit ist eine Kanalöffnung vorgesehen, über welche ein in dem endoskopischen Instrument ausgebildeter Fluidkanal zur Umgebung hin offen ist. Ist die Kopfeinheit vollständig, also bis einschließlich dem die Kanalöffnung aufweisenden Bereich derselben, in die Gelenkshöhle eingeführt, kann in dieser angesammelte Flüssigkeit durch die Kanalöffnung hindurch in den Fluidkanal strömen und am proximalen Endbereich des endoskopischen Instruments austreten. Der Austritt von Flüssigkeit indiziert, dass die in der Kopfeinheit vorgesehene Kanalöffnung innerhalb der Gelenkhöhle positioniert ist. Daraufhin kann das Maulteil aus seiner Ruhestellung heraus in Richtung auf den spitz ausgebildeten distalen Endbereich der Kopfeinheit verschwenkt werden, bis es näherungsweise orthogonal zur Kopfeinheit steht. Eine am Maulteil ausgebildete Gewebeeingriffsformation, beispielsweise eine Mehrzahl von Zähnen oder dergleichen, ist dabei in Richtung vom distalen Endbereich der Kopfeinheit weg, also in Richtung proximal orientiert. Durch Zurückziehen des endoskopischen Instruments, also eine Herausziehbewegung der Kopfeinheit aus der Gelenkhöhle, wird das seitlich von der Kopfeinheit abstehende Maulteil mit seiner Gewebeeingriffsformation in Kontakt mit der Innenseite der Synovia gebracht, was durch einen erhöhten Herausziehwiderstand erkennbar wird. Durch Zurückverschwenken des Maulteils in Richtung auf seine Ruhestellung zu kann ein Teil der Synovia als Biopsat abgetrennt werden und in der Ruhestellung des Maulteils bei dann weiter fortgesetztem Herausziehen des endoskopischen Instruments in der Kopfeinheit gehalten werden.

Mit einem derartigen endoskopischen Instrument besteht die Möglichkeit, eine Biopsie, beispielsweise eine Synovialbiopsie, vorzunehmen, ohne dass substantielle Verletzungen im Gewebe erzeugt werden bzw. größere Schnitte vorgesehen werden müssen, um das endoskopische Instrument in das Gewebe bzw. den zu untersuchenden Bereich einführen zu können. Des weiteren kann durch die retrograde Arbeitsweise und die dabei genutzte Indizierung der Positionierung der Kopfeinheit über den Austritt von Flüssigkeit auf zusätzliche optische Instrumente, welche die exakte Positionierung der Kopfeinheit beispielsweise innerhalb einer Gelenkhöhle zeigen, verzichtet werden.

Die US 5,569,299 A offenbart eine endoskopische Biopsiezange mit einer an einer Betätigungseinheit getragenen Kopfeinheit. Ein durch die Betätigungseinheit zur Verschwenkung antreibbares Maulteil ist auf einem feststehenden Kopfteil schwenkbar getragen. Das einen distalen Spitzenbereich der Kopfeinheit bereitstellende Maulteil übergreift in seiner eingeschwenkten Ruhestellung einen distalen Endbereich des feststehenden Kopfteils. Bei Ver schwenkung in seine Arbeitsstellung wird das Maulteil vom distalen Endbereich des feststehenden Kopfteils weg verschwenkt, so dass am Maulteil vorgesehe und eine Gewebeeingriffsformation bereitstellende Zähne in Richtung distal orientiert sind. Mit dem in dieser Positionierung auf den Bereich eines Gewebes, von welchem eine Probe zu entnehmen ist, positionierten Maulteil kann dieses in Eingriff mit dem Gewebe gebracht werden, so dass beim Zurückverschwenken des Maulteils in seine Ruhestellung, also in Richtung auf den distalen Endbereich des feststehenden Kopfteils zu, eine Gewebeprobe abgetrennt werden kann. In dem feststehenden Kopfteil sind Öffnungen vorgesehen, durch welche ein Fluidkanal im Bereich der Kopfeinheit zur Umgebung offen ist.

Es ist die Aufgabe der vorliegenden Erfindung, ein derartiges endoskopisches Instrument so weiterzubilden, dass eine verbesserte Funktionalität bei der retrograden Biopsie erreicht wird.

Erfindungsgemäß wird diese Aufgabe gelöst durch ein endoskopisches Instrument für die retrograde Biopsie, insbesondere Synovialbiopsie, umfassend eine Kopfeinheit mit einem distalen Spitzenbereich zur Durchdringung von Gewebe, einem Maulteil mit einer Gewebeeingriffsformation zur Biopsatabtrennung, wobei das Maulteil von einer Ruhestellung in eine Arbeitsstellung in Richtung auf den distalen Spitzenbereich zu und zurück verschwenkbar ist, wobei in der Arbeitsstellung die Gewebeeingriffsformation im Wesentlichen von dem distalen Spitzenbereich weg orientiert ist, sowie einem im Bereich der Kopfeinheit zur Umgebung offenen ersten Fluidkanalbereich.

Dabei ist weiter vorgesehen, dass der erste Fluidkanalbereich wenigstens bei in der Ruhestellung positioniertem Maulteil über wenigstens eine im Maulteil vorgesehene Kanalöffnung zur Umgebung offen ist.

Bei dem erfindungsgemäßen Aufbau eines derartigen endoskopischen Instruments zur retrograden Biopsie, insbesondere Synovialbiopsie, kann durch die Integration der Flüssigkeit aus dem zu untersuchenden Körper, also beispielsweise einer Gelenkshöhle, aufnehmenden oder in diese einspeisenden Kanalöffnung im Bereich des Maulteils die Baulänge der Kopfeinheit und damit der beispielsweise in eine Gelenkshöhle zur Durchführung einer retrograden Biopsie einzuführende Längenabschnitt des endoskopischen Instruments verringert werden. Dies bedeutet eine geringere Eindringtiefe in den zu untersuchenden Körper und damit eine entsprechend geringere Belastung des untersuchten Körpers bzw. auch in eine entsprechend geringerer Gefahr der Erzeugung von Verletzungen in dem zu untersuchenden Bereich.

Um bei der Durchführung einer Biopsie die Kopfeinheit zuverlässig am zu untersuchenden Bereich positionieren und das Maulteil zwischen seiner Ruhestellung und seiner Arbeitsstellung verschwenken zu können, wird eine Betätigungseinheit mit einem in seinem distalen Endbereich die Kopfeinheit tragenden Betätigungsschaft und einem zur Verschwenkung des Maulteils im Betätigungsschaft bewegbar aufgenommenen Betätigungsorgan vorgeschlagen.

Zur Betätigungskraftübertragung zwischen dem Betätigungsorgan und dem an der Kopfeinheit vorgesehenen Maulteil wird vorgeschlagen, dass die Kopfeinheit ein am distalen Endbereich des Betätigungsschafts vorzugsweise lösbar angebrachtes erstes Kopfteil und ein durch das Betätigungsorgan bezüglich des ersten Kopfteils in einer Verschieberichtung verschiebbares zweites Kopfteil umfasst, wobei das Maulteil mit dem ersten Kopfteil um eine erste Schwenkachse verschwenkbar verbunden ist und mit dem zweiten Kopfteil um eine zweite Schwenkachse schwenkbar verbunden ist. Somit kann durch Erzeugung einer Relativbewegung zwischen den beiden Kopfteilen aufgrund der Ankopplung des Maulteils an jedes dieser Kopfteile die Verschwenkung des Maulteils erzwungen werden bzw. eine Verschiebebewegung in eine Schwenkbewegung umgesetzt werden.

Eine zuverlässige Erzeugung bzw. Übertragung einer Betätigungskraft in beiden Betätigungsrichtungen kann bei einem vorteilhaften Aufbau dadurch gewährleistet werden, dass das Betätigungsorgan eine in ihrem distalen Endbereich mit dem zweiten Kopfteil zur gemeinsamen Verschiebung in der Verschieberichtung verbundene und in ihrem proximalen Endbereich durch eine Manipulationsanordnung beaufschlagbare Betätigungsstange umfasst.

Eine Beeinträchtigung der Bewegung der Kopfeinheit durch das zu durchdringende Gewebe hindurch kann dadurch vermieden bzw. minimiert werden, dass im zweiten Kopfteil eine durch das Maulteil in der Ruhestellung im Wesentlichen abgeschlossene Maulteilaufnahmeöffnung ausgebildet ist, so dass das Maulteil zumindest bereichsweise in dieser Öffnung aufgenommen bzw. in der Umfangskontur des zweiten Kopfteils und somit der Kopfeinheit versenkt gehalten werden kann.

Der distale Spitzenbereich kann am zweiten Kopfteil vorgesehen sein und im Wesentlichen durch den bzw. in dem distalen Endbereich des zweiten Kopfteils und somit auch des endoskopischen Instruments vorgesehen sein.

Um in der Ruhestellung des Maulteils sicherstellen zu können, dass ein Fluidaustausch im Wesentlichen nur über die im Maulteil vorgesehene Kanalöffnung erfolgen kann, wird vorgeschlagen, dass der erste Fluidkanalbereich in der Ruhestellung des Maulteils im Wesentlichen durch das erste Kopfteil, das zweite Kopfteil und das Maulteil umgrenzt ist und über die wenigstens eine Kanalöffnung im Maulteil zur Umgebung offen ist.

Weiterhin kann vorgesehen sein, dass das zweite Kopfteil in der Ruhestellung des Maulteils in der Verschieberichtung am ersten Kopfteil abgestützt oder abstützbar ist. Somit kann sichergestellt werden, dass bei der Eindringbewegung und der dabei auf das zweite Kopfteil einwirkenden Reaktionskraft dieses in der Verschieberichtung fest abgestützt ist und keine undefinierten Relativbewegungen zwischen dem ersten Kopfteil und dem zweiten Kopfteil entstehen.

Zur Anbindung des in der Kopfeinheit bereitgestellten ersten Fluidkanalbereichs an weitere fluidleitende Systembereiche wird vorgeschlagen, dass das erste Kopfteil in einem proximalen Endbereich der Kopfeinheit eine Kopplungsanordnung zur Kopplung mit dem Betätigungsschaft in einem distalen Endbereich der Betätigungseinheit aufweist, wobei der erste Fluidkanalbereich in der Kopplungsanordnung zu einem in der Betätigungseinheit ausgebildeten zweiten Fluidkanalbereich offen ist.

Bei dieser Ausgestaltung kann der zweite Fluidkanalbereich in baulich einfacher Weise wenigstens bereichsweise zwischen einer Innenoberfläche des Betätigungsschafts und einer Außenoberfläche des Betätigungsorgans gebildet sein.

Um einen Fuidaustausch mit außerhalb des zu untersuchenden Körpers liegenden Systembereichen zu ermöglichen, wird vorgeschlagen, dass die Betätigungseinheit in ihrem proximalen Endbereich einen zum zweiten Fluidkanalbereich offenen Kanalabzweigungsbereich zum Anschluss an eine Fluidquelle oder/und ein Fluidreservoir aufweist.

Der Austritt von über die beiden Fluidkanalbereiche geleitetem Fluid aus der Betätigungseinheit sowie auch der Eintritt von Verunreinigungen insbeson - dere in den zweiten Fluidkanalbereich können dadurch verhindert werden, dass die Betätigungseinheit in ihrem proximalen Endbereich eine den zweiten Fluidkanalbereich abschließende fluiddichte Durchführung für das Betätigungsorgan aufweist. Dabei kann der Kanalabzweigungsbereich bzw. alternativ oder zusätzlich auch wenigstens ein Teil der Durchführung in einem mit dem Betätigungsschaft in dessen proximalen Endbereich verbundenen Anschlusselement vorgesehen sein.

Die vorliegende Erfindung wird nachfolgend anhand der beiliegenden Figu - ren detailliert beschrieben. Es zeigt:
- Fig. 1: in perspektivischer Ansicht ein endoskopisches Instrument, welches beispielsweise zur retrograden Biopsie, insbesondere Synovialbiopsie, eingesetzt werden kann;
- Fig. 2: eine im distalen Endbereich des Instruments der Fig. 1 vorgesehene Kopfeinheit mit einem in einer Arbeitsstellung positionierten Maulteil;
- Fig. 3: die Kopfeinheit der Fig. 2 in anderer perspektivischer Ansicht;
- Fig. 4: die Kopfeinheit des in Fig. 1 dargestellten Instruments mit in einer Ruhestellung positioniertem Maulteil;
- Fig. 5: ein erstes Kopfteil der in den Fig. 2 bis 4 dargestellten Kopfeinheit;
- Fig. 6: ein zweites Kopfteil der in den Fig. 2 bis 4 dargestellten Kopfeinheit;
- Fig. 7: das Maulteil der in den Fig. 2 bis 4 dargestellten Kopfeinheit;
- Fig. 8: das in Fig. 6 dargestellte zweite Kopfteil mit dem daran schwenkbar getragenen Maulteil und einer mit dem zweiten Kopfteil gekoppelten Betätigungsstange in Draufsicht;
- Fig. 9: die in Fig. 8 dargestellte Baugruppe in Seitenansicht;
- Fig. 10: die in Fig. 4 dargestellte Kopfeinheit mit einer Betätigungsstange im Längsschnitt;
- Fig. 11: die mit den Teilen der Fig. 5 bis 7 aufgebaute Kopfeinheit bei in der Arbeitsstellung positioniertem Maulteil in Seitenansicht;
- Fig. 12: einen Teil einer Betätigungseinheit des endoskopischen Instruments der Fig. 1 im Längsschnitt.

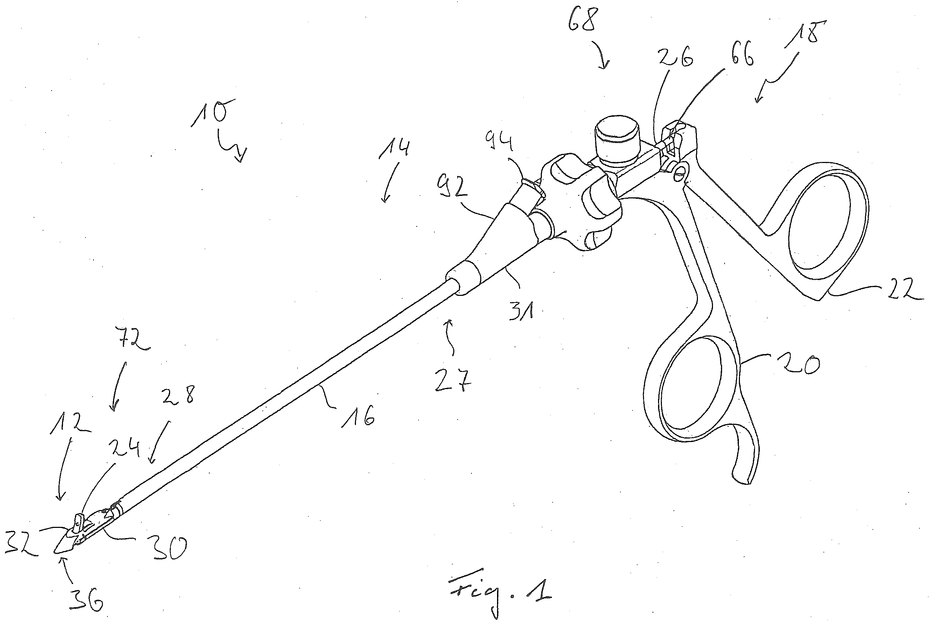

In Fig. 1 ist ein insbesondere für die retrograde Synovialbiopsie einsetzba - res endoskopisches Instrument allgemein mit 10 bezeichnet. Das endoskopische Instrument 10 umfasst eine in den durch Gewebeentnahme zu untersuchenden Bereich eines Körpers einzuführende Kopfeinheit 12 sowie eine allgemein mit 14 bezeichnete Betätigungseinheit. Die Betätigungseinheit 14 umfasst einen die Kopfeinheit 12 tragenden, hohlen bzw. rohrartigen Betätigungsschaft 16 sowie eine Manipulationsanordnung 20 mit zwei scherenartig bezüglich einander schwenkbaren Handgriffteilen 20, 22. Durch Verschwenkung der beiden Handgriffteile 20, 22 wird eine Betätigungskraft zur Verschwenkung eines Maulteils 24 der Kopfeinheit 24 erzeugt, die über ein nachfolgend noch erläutertes und auch den Betätigungsschaft 16 durchsetzendes, beispielsweise als Betätigungsstange ausgebildetes Betätigungsorgan 26 auf das Maulteil 24 übertragen wird.

Der Betätigungsschaft 16 ist in seinem proximalen Endbereich 27 über ein Anschlusselement 31 an der Manipulationsanordnung 18 getragen. Über das Anschlusselement 31 kann in nachfolgend detailliert beschriebener Art und Weise Flüssigkeit, beispielsweise Gewebsflüssigkeit, aus dem durch Gewebeentnahme zu untersuchenden Bereich abgeführt werden bzw. Flüssigkeit in diesen Bereich eingeleitet werden.

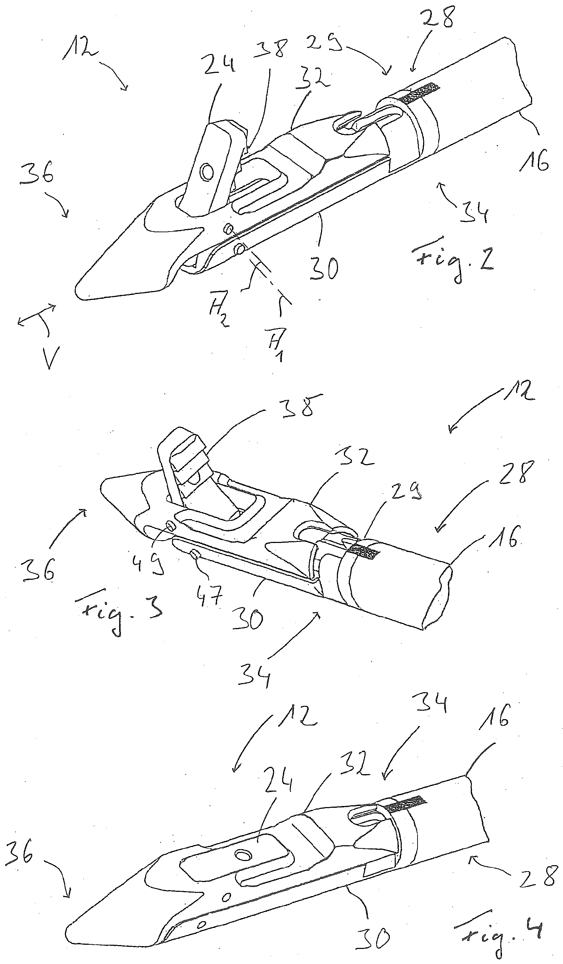

Mit Bezug auf die Fig. 2 bis 4 wird nachfolgend die grundsätzliche Funktionalität des in Fig. 1 dargestellten endoskopischen Instruments 10 bei der Durchführung einer Biopsie erläutert.

Die in Fig. 2 erkennbare Kopfeinheit 12 umfasst ein am distalen Endbereich 28 des Betätigungsschafts 16 beispielsweise unter Einsatz einer nach Art eines Bajonettverschlusses aufgebauten Kopplungsanordnung 29 lösbar angebrachtes bzw. anbringbares erstes Kopfteil 30. Die Kopfeinheit 12 umfasst ferner ein in einer Verschieberichtung V, welche beispielsweise einer Längserstreckungsrichtung des Betätigungsschafts 16 entsprechen kann, bezüglich des ersten Kopfteils 30 hin und her verschiebbares zweites Kopfteil 32. Während der proximale Endbereich 34 der Kopfeinheit 12 am ersten Kopfteil 30 vorgesehen und mit dem distalen Endbereich 28 des Betätigungsschafts 16 koppelbar ist, ist der distale Endbereich 36 der Kopfeinhiet 12 am zweiten Kopfteil 32 vorgesehen, so dass bei Verschiebebewegung der beiden Kopfteile 30, 32 bezüglich einander auch der distale Endbereich 36 und der proximale Endbereich 34 der Kopfeinheit 12 sich in der Verschieberichtung V bezüglich einander verschieben. Im distalen Endbereich 36 ist die Kopfeinheit 12 spitz ausgebildet, so dass sie nach Art eines Trokars durch das Gewebe eines Körpers unter Erzeugung eines Durchgangs für das endoskopische Instrument 10 hindurch geführt werden kann.

Das Maulteil 24 ist mit dem ersten Kopfteil 30 um eine zur Verschieberichtung V im Wesentlichen orthogonal stehende erste Schwenkachse A₁ schwenkbar verbunden. Gleichermaßen ist das Maulteil 24 mit dem zweiten Kopfteil 32 um eine zur Verschieberichtung V beispielsweise ebenfalls im Wesentlichen orthogonal stehende zweite Schwenkachse A₂ schwenkbar verbunden, wobei die beiden Schwenkachsen A₁ und A₂ zueinander vorzugsweise im Wesentlichen parallel und exzentrisch liegen.

Bei Verschiebung der beiden Kopfteile 30, 32 bezüglich einander in der Verschieberichtung V wird durch die schwenkbare Anbindung des Maulteils 24 an die beiden Kopfteile 30, 32 dieses zwischen einer in den Fig. 1 bis 3 erkennbaren Arbeitsstellung und einer in Fig. 4 dargestellten Ruhestellung hin- und her verschwenkt. Bei Verschwenkung aus der Ruhestellung der Fig. 4 in die beispielsweise in Fig. 3 erkennbare Arbeitsstellung verschwenkt das Maulteil 24 in Richtung auf den spitz ausgebildeten distalen Endbereich 36 zu, bis in der Arbeitsstellung das Maulteil 24 vorteilhafterweise näherungs - weise orthogonal zur Verschieberichtung V bzw. auch zur Längserstreckungsrichtung des Betätigungsschafts 16 bzw. auch der Kopfeinheit 12 steht. Eine am Maulteil 24 vorgesehene Gewebeeingriffsformation 38 ist in der Arbeitsstellung in Richtung von dem spitz ausgebildeten distalen Endbe - reich 36 weg orientiert und liegt in der in Fig. 4 dargestellten Ruhestellung des Maulteils 24 im Inneren der Kopfeinheit 12 verborgen, so dass das Maulteil grundsätzlich rückwärts schneidend eingesetzt werden kann. Es sei hier darauf hingewiesen, dass die Gewebeeingriffsformation 38 am Maulteil 24 in verschiedenster Weise ausgeführt sein kann. So kann diese beispiels - weise, wie insbesondere in Fig. 3 erkennbar, nach Art einer Widerhaken - stanze ausgeführt sein. Sie kann auch löffelartig ausgebildet sein mit einer am Löffelrand umlaufenden Widerhakenstanze oder/und als Löffelstanze mit einem Mausezahn.

Ist die Kopfeinheit 12 bei in der Ruhestellung positioniertem Maulteil 24 in den zu untersuchenden Körperbereich, also beispielsweise eine Gelenkshöhle, eingeführt, kann durch Betätigung der Manipulationsanordnung 18 das Maulteil 24 aus seiner Ruhestellung heraus in die Arbeitsstellung verschwenkt werden und dann durch Bewegen des endoskopischen Instruments 10 in Richtung aus dem zu untersuchenden Körper heraus mit seiner Gewebeeingriffsformation 38 in Kontakt mit dem zu untersuchenden Gewebe, also beispielsweise der Innenseite der Synovia, gebracht werden. Beim Zurückverschwenken des Maulteils 24 in Richtung vom distalen Endbereich 36 weg in seine Ruhestellung entnimmt das Maulteil 24 mit seiner Gewebeeingriffsformation 38 Gewebe als Biopsat und hält dieses bei dann in der Ruhestellung positioniertem Maulteil 24 im Inneren der Kopfeinheit 12, so dass das so entnommene Biopsat durch Herausziehen des endoskopischen Instruments 10 aus dem untersuchten Körper herausgeführt werden kann und durch erneutes Verschwenken des Maulteils 24 in seine Arbeitsstellung außerhalb des Körpers zur weiteren Untersuchung aus der Kopfeinheit 12 bzw. vom Maulteil 24 entfernt werden kann.

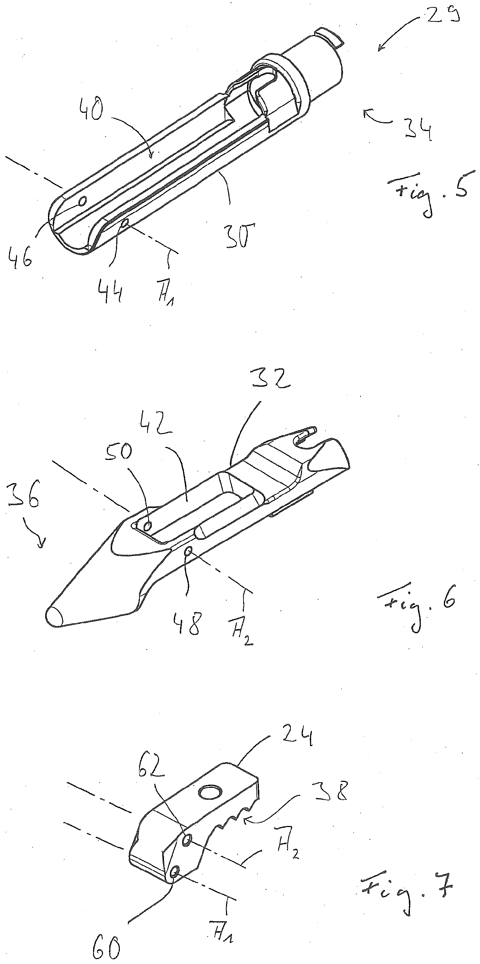

Die zum Aufbau der Kopfeinheit 12 eingesetzten Bauteile sind in den Fig. 5 bis 7 detailliert dargestellt. So zeigt die Fig. 5 in perspektivischer Ansicht das erste Kopfteil 30 mit seiner im Wesentlichen auch den proximalen Endbereich 34 der Kopfeinheit 12 bereitstellenden, zur Ankopplung an den Betätigungsschaft 16 vorgesehenen Kopplungsanordnung 29. Wie bereits ausgeführt, kann die Verbindung zwischen dem ersten Kopfteil 30 und dem Betätigungsschaft 16 nach Art eines Bajonettverschlusses hergestellt werden, so dass einerseits eine stabile Halterung dieser beiden Baugruppen anein - ander gewährleistet ist, andererseits diese beiden Baugruppen insbesondere zur Durchführung von Reinigungsvorgängen leicht voneinander gelöst werden können.

Das erste Kopfteil 30 ist im Wesentlichen als rohrartiges bzw. schalenartiges und in einem Seitenbereich und einem Endbereich offenes Bauteil ausgeführt und stellt einen zumindest bereichsweise durch das in Fig. 6 erkennbare zweite Kopfteil 32 abgeschlossenen Innenvolumenbereich 40 bereit. Am in Fig. 6 erkennbaren zweiten Kopfteil 32 ist neben dem im Wesentlichen spitz zulaufend ausgebildeten distalen Endbereich 36 der Kopfeinheit 12 eine Maulteilaufnahmeöffnung 42 ausgebildet. Bei in der Ruhestellung positioniertem Maulteil 24 ist diese Maulteilaufnahmeöffnung 42 im Wesentlichen vollständig durch das in Fig. 7 dargestellte Maulteil 24 abgedeckt bzw. verschlossen, wobei in der Ruhestellung das Maulteil 24 so bezüglich des zweiten Kopfteils 24 positoniert ist, dass es über dessen Außenumfangskontur nicht hervorsteht (siehe Fig. 4). Dadurch ist gewährleistet, dass das Eindringen der Kopfeinheit 12 in das Gewebe eines Körpers nicht durch über die Außenkontur des zweiten Kopfteils 32 hervorstehende Abschnitte des Maulteils 24 beeinträchtigt ist.

Sowohl im ersten Kopfteil 30, als auch im zweiten Kopfteil 32, als auch im Maulteil 24 sind jeweilige einander paarweise zugeordnete Öffnungen 44, 46 bzw. 48, 50 bzw. 60, 62 vorgesehen. Zur Schwenkankopplung des Maulteils 24 an das erste Kopfteil 30 kann ein Schwenkstift 47 die Öffnungen 44, 46 und 60 durchsetzend angeordnet werden, so dass das Maulteil 24 am ersten Kopfteil 30 um die Schwenkachse A₁ schwenkbar ist. Zur Schwenkanbindung an das zweite Kopfteil 32 kann ein Schwenkstift 49 die Öffnungen 48, 50 und 62 durchsetzend angeordnet werden, so dass das Maulteil 24 am zweiten Kopfteil 32 um die zweite Schwenkachse A₂ schwenkbar ist. Durch Relativverlagerung der beiden Kopfteile 30, 32 bezüglich einander verschie - ben sich auch die Schwenkachsen A₁ und A₂ bezüglich einander, so dass die Verschiebung der beiden Kopfteile 30, 32 bezüglich einander die Verschwenkung des Maulteils 24 zwischen seiner Ruhestellung und seiner Arbeitsstellung erzwingt. Im Verlaufe dieser Schwenkbewegung wird aufgrund des fest vorgegebenen Abstands der beiden Schwenkachsen A₁ und A₂ bezüglich einander das zweite Kopfteil 32 bei seiner Verschiebebewegung in der Richtung V eine geringfügige seitliche Auslenkbewegung bezüglich des ersten Kopfteils 30 erfahren, was jedoch dem nicht entgegensteht, dass diese beiden Kopfteile 30, 32 im Wesentlichen bezüglich einander verschoben werden.

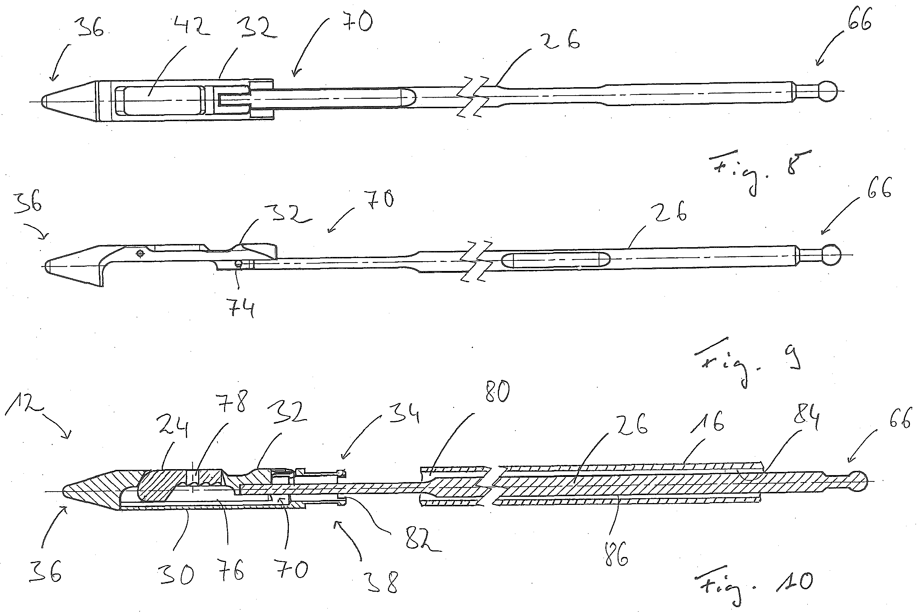

Diese Relativverschiebung der beiden Kopfteile 30, 32 bezüglich einander wird durch die den Betätigungsschaft 16 durchsetzende und in den Fig. 8 bis 10 erkennbare Betätigungsstange 26 bewirkt. Diese ist in ihrem proximalen Endbereich 66 im proximalen Endbereich 68 des endoskopischen Instruments 10 mit der Manipulationsanordnung 18, insbesondere dem Handgriffteil 22, gekoppelt und ist in ihrem distalen Endbereich 70 im distalen Endbereich 72 des endoskopischen Instruments 10 beispielsweise durch einen Kopplungsstift 74 mit dem zweiten Kopfteil 32 zur gemeinsamen Verschiebung in der Verschieberichtung V gekoppelt. Durch Verschwenkung des Handgriffteils 22 bezüglich des Handgriffteils 20 wird, je nach Schwenkrichtung, die Betätigungsstange 26 in Richtung distal oder in Richtung proximal verschoben, wobei eine Verschiebung der Betätigungsstange 26 in Richtung distal eine entsprechende Verschiebung des zweiten Kopfteils 32 in Richtung distal bewirkt und dabei eine Verschwenkung des Maulteils 24 in Richtung Arbeitsstellung hervorruft. Eine Verschiebung der Betätigungsstange 26 in Richtung proximal bewegt das zweite Kopfteil 32 gleichermaßen in Richtung proximal und erzwingt somit eine Verschwenkung des Maulteils 24 in Richtung vom distalen Endbereich 36 der Kopfeinheit 12 weg in seine Ruhestellung.

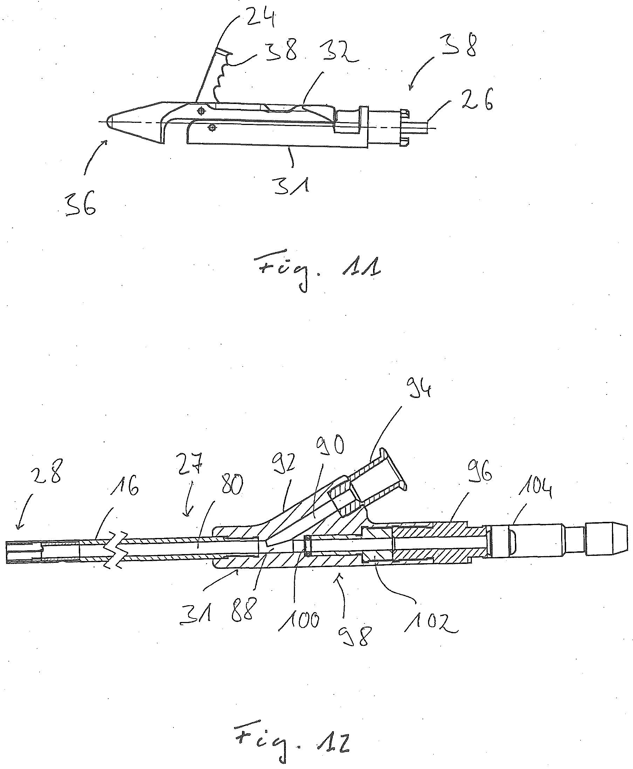

In der Kopfeinheit 12 ist ein in Fig. 10 allgemein mit 76 bezeichneter erster Fluidkanalbereich ausgebildet. Dieser ist im Wesentlichen durch den Innenvolumenbereich 40 des ersten Kopfteils 30 bereitgestellt und ist durch das erste Kopfteil 30 zusammen mit dem zweiten Kopfteil 32 und auch dem Maulteil 24 umgrenzt, insbesondere dann, wenn das Maulteil 24 in seiner Ruhestellung ist. Dabei ist, wie die Fig. 10 dies zeigt, das im Wesentlichen maximal in Richtung proximal verlagerte zweite Kopfteil 32 in der Verschieberichtung V, und zwar in Richtung proximal, am ersten Kopfteil 30 abstützt. Der erste Fluidkanalbereich 76 ist in diesem Zustand über eine im Maulteil 24 ausgebildete Kanalöffnung 78 zur Umgebung im Bereich der Kopfeinheit 12 offen. Befindet sich das Maulteil 24 in seiner Arbeitsstellung, also dem beispielsweise auch in den Fig. 2, 3 und 11 erkennbaren Zustand, ist der erste Fluidkanalbereich 76 nicht bzw. nicht nur über die im Maulteil 24 aus - gebildete Kanalöffnung 78 zur Umgebung hin offen, sondern über im Wesentlichen die ganze im zweiten Kopfteil 32 ausgebildete Maulteilaufnahmeöffnung 42 bzw. den in der Verschieberichtung V auch offenen Volumenbereich 40 des ersten Kopfteils 32.

Der erste Fluidkanalbereich 76 ist im proximalen Endbereich 34 der Kopfeinheit 12 des Weiteren zu einem auch im Inneren des Betätigungsschafts 16 ausgebildeten zweiten Fluidkanalbereich 80 offen. Insbesondere ist im proximalen Endbereich 34 im ersten Körperteil 30 eine im Wesentlichen axial offene Öffnung 82 gebildet, welche zum Innenvolumenbereich des Betätigungsschafts 16 hin offen ist. Der zweite Fluidkanalbereich 80 ist im Wesentlichen zwischen einer Innenoberfläche 84 des Betätigungsschafts 16 und einer Außenoberfläche 86 der Betätigungsstange 26 gebildet und somit in diesem Bereich als in Richtung des Betätigungsschafts 16 langgestrecktes ringartiges Volumen ausgebildet.

Im proximalen Endbereich 27 des Betätigungsschafts 16 ist der zweite Fluidkanalbereich 80 fortgesetzt durch eine in der Längsrichtung des Betätigungsschafts 16 sich erstreckende kanalartige Öffnung 88 im Anschlusselement 31. In diese kanalartige Öffnung 88 mündet ein Kanalabzweigungsbereich 90 ein, der in einem seitlichen Ansatz 92 des Anschlusselements 31 ausgebildet ist. Dieser seitliche Ansatz 92 bzw. der darin ausgebildete Kanalabzweigungsbereich 90 kann über ein weiteres Anschlusselement 94, beispielsweise Luer-Anschlusselement, in Verbindung gebracht werden mit einer Fluidquelle bzw. einem Fluidreservoir, beispielsweise einer Spritze. Somit wird es möglich, über den im Bereich des Maulteils 24 offenen ersten Fluidkanalbereich 76, den zweiten Fluidkanalbereich 80 und den Kanalabzweigungsbereich 90 Gewebsflüssigkeit aus dem zu untersuchenden Bereich aufzunehmen bzw. abzuziehen. Tritt über den Kanalabzweigungsbereich 90 derartige Flüssigkeit aus, so ist dies ein Zeichen dafür, dass die Kopfeinheit 12 und das daran vorgesehene Maulteil 24 in dem zu untersuchenden Bereich positioniert ist. Andererseits wird es möglich, über die Fluidkanalbereiche 76, 80 Flüssigkeit in den zu untersuchenden Bereich ein - zuspritzen, beispielsweise zur Behandlung oder lokalen Betäubung.

In dem Anschlusselement 31 ist eine allgemein mit 98 bezeichnete fluiddichte Durchführung für die Betätigungsstange 26 gebildet. Diese umfasst ein beispielsweise als O-Ring ausgebildetes Dichtungselement 100. Dieses ist axial zwischen einer stufenartigen Erweiterung im Anschlusselement 31 und einem in dieses eingesetzten Einsatzteil 102 gehalten. Das Einsatzteil 102 wiederum ist zusammen mit einem Zwischenteil 96 am Anschlusselement 31 gehalten, derart, dass die Betätigungsstange 26 durch das Anschlusselement 31, das Einsatzteil 102 und das Zwischenteil 96 hindurch bis in den Bereich eines in der Manipulationsanordnung 18 zu verankernden weiteren Einsatzteils 104 bzw. durch dieses hindurchgeführt werden kann, so dass der proximale Endbereich 66 mit dem Handgriffteil 22 gekoppelt werden kann.

Mit dem vorangehend beschriebenen Aufbau eines endoskopischen Instruments wird die Durchführung einer retrograden Biopsie ermöglicht, insbesondere zur Entnahme von Synovialgewebe. Dabei kann der Austritt von Gewebsflüssigkeit als Indikator für das Erreichen des zu untersuchenden Bereichs genutzt werden. Da diese Gewebsflüssigkeit im Bereich der im Maulteil ausgebildeten Kanalöffnung in die Kopfeinheit eintreten kann, ist ein übermäßig weites Eindringen mit der Kopfeinheit in den zu untersuchenden Bereich, insbesondere den Bereich eines Gelenks, nicht erforderlich.

Da das erfindungsgemäße endoskopische Instrument aus verschiedenen leicht voneinander zu entkoppelnden Baugruppen aufgebaut ist, insbesondere die Kopfeinheit in einfacher Weise mit dem Betätigungsschaft gekoppelt bzw. von diesem entkoppelt werden kann, sind die einzelnen Baugruppen bzw. Bestandteile des erfindungsgemäßen endoskopischen Instruments in einfacher Weise, gleichwohl jedoch mit hoher Zuverlässigkeit zu reinigen bzw. zu desinfizieren. Es ist selbstverständlich, dass auch zu diesem Zweck die für den Aufbau des endoskopischen Instruments genutzten Materialien eine ausreichende Korrosionsbeständigkeit gewährleisten müssen, ebenso wie eine ausreichende Biokompatibilität und mechanische Festigkeit.

Das endoskopische Instrument kann mit sehr kompakter Baugröße bereitgestellt werden und vermeidet somit größere Beeinträchtigungen des durch dieses durchdrungenen Gewebes. Dazu trägt insbesondere auch bei, dass im Bereich des Betätigungsschafts der zweite Fluidkanalbereich nicht durch separat dafür vorzusehende Kanalelemente bereitgestellt werden muss, sondern der Innenvolumenbereich des Betätigungsschafts auch zur Fluidführung genutzt werden kann. Dies gewährleistet des Weiteren eine zuverlässige Fluiddurchströmung ohne wesentlichem Fluidströmungswiderstand, vermeidet jedoch gleichzeitig übergroße Totvolumina. Bereits eine vergleichsweise geringe Menge von Gelenksflüssigkeit kann daher durch Austritt am distalen Endbereich erkannt und als Indikator für die Positionierung in dem zur Untersuchung vorgesehenen Bereich genutzt werden.

## Patentansprüche

1. Endoskopisches Instrument für die retrograde Biopsie, insbesondere Synovialbiopsie, umfassend eine Kopfeinheit (12) mit
- einem distalen Spitzenbereich (36) zur Durchdringung von Gewebe,
- einem Maulteil (24) mit einer Gewebeeingriffsformation (38) zur Biopsatabtrennung, wobei das Maulteil (24) von einer Ruhestellung in eine Arbeitsstellung in Richtung auf den distalen Spitzenbereich (37) zu und zurück verschwenkbar ist, wobei in der Arbeitsstellung die Gewebeeingriffsformation (38) im Wesentlichen von dem distalen Spitzenbereich (37) weg orientiert ist,
- einem im Bereich der Kopfeinheit (12) zur Umgebung offenen ersten Fluidkanalbereich (76),
**dadurch gekennzeichnet, dass** der erste Fluidkanalbereich (76) wenigstens bei in der Ruhestellung positioniertem Maulteil (24) über wenigstens eine im Maulteil (24) vorgesehene Kanalöffnung (78) zur Umgebung offen ist.

2. Endoskopisches Instrument nach Anspruch 1, **gekennzeichnet durch** eine Betätigungseinheit (14) mit einem in seinem distalen Endbereich (28) die Kopfeinheit (12) tragenden Betätigungsschaft (16) und einem zur Verschwenkung des Maulteils (24) im Betätigungsschaft (16) bewegbar aufgenommenen Betätigungsorgan (26).

3. Endoskopisches Instrument nach Anspruch 2,
**dadurch gekennzeichnet, dass** die Kopfeinheit (12) ein am distalen Endbereich (28) des Betätigungsschafts (16) vorzugsweise lösbar angebrachtes erstes Kopfteil (30) und ein durch das Betätigungsorgan (26) bezüglich des ersten Kopfteils (30) in einer Verschieberichtung (V) verschiebbares zweites Kopfteil (32) umfasst, wobei das Maulteil (24) mit dem ersten Kopfteil (30) um eine erste Schwenkachse (A₁) verschwenkbar verbunden ist und mit dem zweiten Kopfteil um eine zweite Schwenkachse (A₂) schwenkbar verbunden ist.

4. Endoskopisches Instrument nach Anspruch 3,
**dadurch gekennzeichnet, dass** das Betätigungsorgan (26) eine in ihrem distalen Endbereich (70) mit dem zweiten Kopfteil (32) zur gemeinsamen Verschiebung in der Verschieberichtung (V) verbundene und in ihrem proximalen Endbereich (66) durch eine Manipulationsanordnung (18) beaufschlagbare Betätigungsstange (26) umfasst.

5. Endoskopisches Instrument nach Anspruch 3 oder 4,
**dadurch gekennzeichnet, dass** im zweiten Kopfteil (32) eine durch das Maulteil (24) in der Ruhestellung im Wesentlichen abgeschlossene Maulteilaufnahmeöffnung (24) ausgebildet ist oder/und dass der distale Spitzenbereich (36) am zweiten Kopfteil (32) vorgesehen ist.

6. Endoskopisches Instrument nach Anspruch 5,
**dadurch gekennzeichnet, dass** der erste Fluidkanalbereich (76) in der Ruhestellung des Maulteils (24) im Wesentlichen durch das erste Kopfteil (30), das zweite Kopfteil (32) und das Maulteil (24) umgrenzt ist und über die wenigstens eine Kanalöffnung (78) im Maulteil (24) zur Umgebung offen ist.

7. Endoskopisches Instrument nach einem der Ansprüche 3 bis 6,
**dadurch gekennzeichnet, dass** das zweite Kopfteil (32) in der Ruhestellung des Maulteils (24) in der Verschieberichtung (V) am ersten Kopfteil (30) abgestützt oder abstützbar ist.

8. Endoskopisches Instrument nach einem der Ansprüche 3 bis 7,
**dadurch gekennzeichnet, dass** das erste Kopfteil (30) in einem proximalen Endbereich der Kopfeinheit (34) eine Kopplungsanordnung (29) zur Kopplung mit dem Betätigungsschaft (16) in einem distalen Endbereich (72) der Betätigungseinheit (14) aufweist, wobei der erste Fluidkanalbereich (76) in der Kopplungsanordnung (29) zu einem in der Betätigungseinheit (14) ausgebildeten zweiten Fluidkanalbereich (80) offen ist.

9. Endoskopisches Instrument nach Anspruch 8,
**dadurch gekennzeichnet, dass** der zweite Fluidkanalbereich (80) wenigstens bereichsweise zwischen einer Innenoberfläche (84) des Betätigungsschafts (16) und einer Außenoberfläche (86) des Betätigungsorgans (26) gebildet ist.

10. Endoskopisches Instrument nach Anspruch 8 oder 9,
**dadurch gekennzeichnet, dass** die Betätigungseinheit (14) in ihrem proximalen Endbereich einen zum zweiten Fluidkanalbereich (80) offenen Kanalabzweigungsbereich (90) zum Anschluss an eine Fluidquelle oder/und ein Fluidreservoir aufweist.

11. Endoskopisches Instrument nach einem der Ansprüche 8 bis 10,
**dadurch gekennzeichnet, dass** die Betätigungseinheit (14) in ihrem proximalen Endbereich eine den zweiten Fluidkanalbereich (80) abschließende fluiddichte Durchführung (98) für das Betätigungsorgan (26) aufweist.

12. Endoskopisches Instrument nach Anspruch 10 oder 11,
**dadurch gekennzeichnet, dass** der Kanalabzweigungsbereich (90) oder/und wenigstens ein Teil der Durchführung (98) in einem mit dem Betätigungsschaft (16) in dessen proximalen Endbereich (27) verbundenen Anschlusselement (31) vorgesehen ist.

## Claims

1. Endoscopic instrument for the retrograde biopsy, in particular a synovial biopsy, comprising a head unit (12) with
- a distal tip region (36) for penetrating tissue,
- a jaw section (24) with a tissue engagement structure (38) for separating a biopsy material, wherein the jaw section (24) can pivot from a resting position to a working position towards the distal tip region (37) and back, wherein in the working position the tissue engagement structure (38) is substantially oriented away from the distal tip region (37),
- a first fluid channel region (76) open to the environment in the region of the head unit (12),
**characterized in that** the first fluid channel region (76) is open to the environment via at least one channel opening (78) provided at the jaw section (24) at least when the jaw section (24) is in the resting position.

2. Endoscopic instrument according to claim 1, **characterized by** an actuation unit (14) with an actuation shaft (16) carrying the head unit (12) in its distal end region (28) and an actuation element (26) received in the actuation shaft (16) for movement to pivot the jaw section (24).

3. Endoscopic instrument according to claim 2,
**characterized in that** the head unit (12) comprises a first head section (30) which is preferably attached detachably to a distal end section (28) of the actuation shaft (16) and a second head section (32) which can be displaced by the actuation element (26) in a displacement direction (V) in relation to the first head section (30), wherein the jaw section (24) is attached to the first head section (30) to pivot about a first pivot axis (A₁) and is attached to the second head section to pivot about a second pivot axis (A₂).

4. Endoscopic instrument according to claim 3,
**characterized in that** the actuation element (26) comprises an actuation rod (26) attached to the second head section (32) in its distal end region (70) for common displacement in the displacement direction (V) which can be actuated by a manipulation arrangement (18) in its proximal end region (66).

5. Endoscopic instrument according to claim 3 or 4,
**characterized in that** a jaw reception opening (24) substantially closed by the jaw section (24) in the resting position is formed in the second head section (32) or/and **in that** the distal tip section (36) is provided at the second head part (32).

6. Endoscopic instrument according to claim 5,
**characterized in that** in the resting position of the jaw section (24) the first fluid channel region (76) is substantially limited by the first head section (30), the second head section (32) and the jaw section (24) and is open to the environment via the at least one channel opening (78) in the jaw section (24).

7. Endoscopic instrument according to one of claims 3 to 6,
**characterized in that** the second head section (32) is or can be supported in the displacement direction (V) at the first head section (30) in the resting position of the jaw section (24).

8. Endoscopic instrument according to one of claims 3 to 7,
**characterized in that** the first head section (30) comprises a coupling arrangement (29) in a proximal end section of the head unit (34) for coupling with the actuation shaft (16) in a distal end region (72) of the actuation unit (14), wherein the first fluid channel region (76) in the coupling arrangement (29) is open to a second fluid channel region (80) formed in the actuation unit (14).

9. Endoscopic instrument according to claim 8,
**characterized in that** the second fluid channel region (80) is at least partially formed between an inner surface (84) of the actuation shaft (16) and an outer surface (86) of the actuation element (26).

10. Endoscopic instrument according to claim 8 or 9,
**characterized in that** the actuation unit (14) comprises in its proximal end region a channel branch region (90) open to the second fluid channel region (80) for connecting to a fluid source or/and a fluid reservoir.

11. Endoscopic instrument according to one of claims 8 to 10,
**characterized in that** the actuation unit (14) comprises in its proximal end region a fluid-tight duct (98) for the actuation element (26) closing the second fluid channel region (80).

12. Endoscopic instrument according to claim 10 or 11,
**characterized in that** the channel branch region (90) or/and at least part of the duct (98) are provided in a connecting element (31) linked to the actuation shaft (16) in its proximal end region (27).

## Revendications

1. Instrument endoscopique pour la biopsie rétrograde, en particulier la biopsie synovial, comprenant une unité de tête (12) avec
- une région de pointe distale (36) pour pénétrer du tissu,
- une partie de mâchoire (24) avec une formation d'engagement de tissu (38) pour la séparation du matériel biopsié, où la partie de mâchoire (24) peut pivoter d'une position de repos vers une position de travail vers la région de pointe distale (37) et en arrière, où dans la position de travail la formation d'engagement de tissu (38) est essentiellement orientée à l'opposé de la région de pointe distale (37),
- une première région de canal de fluide (76) ouverte vers l'environnement dans la région de l'unité de tête (12),
**caractérisé en ce que** la première région de canal de fluide (76), au moins quand la partie de mâchoire (24) est dans la position de repos, est ouverte vers l'environnement via au moins une ouverture de canal (78) prévue dans la partie de mâchoire (24).

2. Instrument endoscopique selon la revendication 1, **caractérisé par** une unité d'actuation (14) avec une tige d'actuation (16) supportant l'unité de tête (12) dans sa région d'extrémité distale (28) et un membre d'actuation (26) reçu de manière mobile dans la tige d'actuation (16) pour pivoter la partie de mâchoire (24).

3. Instrument endoscopique selon la revendication 2,
**caractérisé en ce que** l'unité de tête (12) comprend une première partie de tête (30) attachée préférablement de manière détachable à la région d'extrémité distale (28) de la tige d'actuation (16) et une deuxième partie de tête (32) déplaçable par le membre d'actuation (26) dans un sens de déplacement (V) par rapport à la première partie de tête (30), la partie de mâchoire (24) étant connectée à la première partie de tête (30) de manière pivotante autour d'un premier axe de pivotement (A₁) et étant connectée à la deuxième partie de tête (32) de manière pivotante autour d'un deuxième axe de pivotement (A₂).

4. Instrument endoscopique selon la revendication 3,
**caractérisé en ce que** le membre d'actuation (26) comprend une barre d'actuation (26) connectée dans sa région d'extrémité distale (70) à la deuxième partie de tête (32) pour un déplacement commun dans le sens de déplacement (V) qui peut être actionnée dans sa région d'extrémité proximale (66) par un arrangement de manipulation (18).

5. Instrument endoscopique selon la revendication 3 ou 4,
**caractérisé en ce qu'**une ouverture de réception de partie de mâchoire essentiellement clôturée par la partie de mâchoire (24) dans la position de repos est formée dans la deuxième partie de tête (32) ou/et **en ce que** la région de pointe distale (36) est prévue à la deuxième partie de tête (32).

6. Instrument endoscopique selon la revendication 5,
**caractérisé en ce que** la première région de canal de fluide (76) est dans la position de repos définie essentiellement par la première partie de tête (30), la deuxième partie de tête (32) et la partie de mâchoire (24) et est ouverte vers l'environnement via ladite au moins une ouverture de canal (78) dans la partie de mâchoire (24).

7. Instrument endoscopique selon une des revendications 3 à 6,
**caractérisé en ce que** la deuxième partie de tête (32) est dans la position de repos de la partie de mâchoire (24) supportée à la première partie de tête (30) ou peut être supportée dans le sens de déplacement (V).

8. Instrument endoscopique selon une des revendications 3 à 7,
**caractérisé en ce que** la première partie de tête (30) comprend dans une région d'extrémité proximale de l'unité de tête (34) un arrangement d'accouplement (29) pour un accouplement avec la tige d'actuation (16) dans une région d'extrémité distale (72) de l'unité d'actuation (14), la première région de canal de fluide (76) étant ouverte dans l'arrangement d'accouplement (29) vers une deuxième région de canal de fluide (80) formée dans l'unité d'actuation (14).

9. Instrument endoscopique selon la revendication 8,
**caractérisé en ce que** la deuxième région de canal de fluide (80) est formée au moins partiellement entre une surface interne (84) de la tige d'actuation (16) et une surface externe (86) du membre d'actuation (26).

10. Instrument endoscopique selon les revendications 8 ou 9,
**caractérisé en ce que** l'unité d'actuation (14) comprend dans sa région d'extrémité proximale une région de branchement de canal (90) ouverte vers la deuxième région de canal de fluide (80) pour une connexion à une source de fluide ou/et un réservoir de fluide.

11. Instrument endoscopique selon une des revendications 8 à 10,
**caractérisé en ce que** l'unité d'actuation (14) comprend dans sa région d'extrémité proximale une conduite (98) étanche au fluide pour le membre d'actuation (26) clôturant la deuxième région de canal de fluide (80).

12. Instrument endoscopique selon les revendications 10 ou 11,
**caractérisé en ce que** la région de branchement de canal (90) ou/et au moins une partie de la conduite (98) sont prévues dans un membre de connexion (31) connecté à la tige d'actuation (16) dans sa région d'extrémité proximale (27).
